Europäisches Patentamt

European Patent Office

Office européen des brevets

(11) Veröffentlichungsnummer: **0 504 873 A1**

# EUROPÄISCHE PATENTANMELDUNG

(21) Anmeldenummer: **92104778.3**

(22) Anmeldetag: **19.03.92**

(51) Int. Cl.5: **C07C 303/38**, C07C 311/03, C07C 311/24

(30) Priorität: **21.03.91 DE 4109243**

(43) Veröffentlichungstag der Anmeldung:
**23.09.92 Patentblatt 92/39**

(84) Benannte Vertragsstaaten:
**AT BE CH DE ES FR GB IT LI NL**

(71) Anmelder: **HOECHST AKTIENGESELLSCHAFT**
**Postfach 80 03 20**
**W-6230 Frankfurt am Main 80(DE)**

(72) Erfinder: **Lachhein, Stephen, Dr.**
**Kiedricher Strasse 18**
**W-6238 Hofheim am Taunus(DE)**
Erfinder: **Dehmer, Klaus, Dr.**
**Sandweg 1**
**W-6233 Kelkheim (Taunus)(DE)**

(54) Verfahren zur Herstellung von N-Alkylsulfonamiden.

(57) Verbindungen der Formel (I)

$R^1\text{-}SO_2\,NH\text{-}R^2$    (I)

worin $R^1$ Alkyl, Alkenyl oder Alkinyl, die gegebenenfalls durch Halogen, Alkoxy oder Alkoxycarbonyl substituiert sind, und $R^2$ Wasserstoff oder $(C_1\text{-}C_4)$Alkyl bedeuten, sind Zwischenprodukte bei der Herstellung von Sulfonylharnstoffherbiziden. Erfindungsgemäß können sie hergestellt werden, in dem man eine Verbindung der Formel $R^1SO_2Cl$    (II) in Gegenwart von etwa äquimolaren Mengen einer Hilfsbase mit etwa äquimolaren Mengen einer Verbindung der Formel $R^2\text{-}NH_2$    (III) in einem halogenierten aromatischen Lösungsmittel umsetzt, anschließend zur Neutralisation des Hydrochlorids der Hilfsbase das Reaktionsgemisch mit wäßrigem Alkalihydroxid oder mit Alkoholaten umsetzt und die Hilfsbase abdestilliert. Bei dem Verfahren werden der sonst übliche große Überschuß an Aminkomponente (II) und Nachteile, die mit der Verwendung wäßriger oder polarer Lösungsmittel verbunden sind, vermieden.

EP 0 504 873 A1

Gegenstand der Erfindung ist ein Verfahren zur Herstellung von Sulfonamiden der Formel (I)

$R^1$-$SO_2$NH-$R^2$     (I)

worin

$R^1$     ($C_1$-$C_6$)Alkyl, ($C_2$-$C_6$)Alkenyl oder ($C_2$-$C_6$)Alkinyl, die gegebenenfalls durch Halogen, ($C_1$-$C_4$)-Alkoxy oder ($C_1$-$C_4$-Alkoxy)-carbonyl substituiert sind, und

$R^2$     Wasserstoff oder ($C_1$-$C_4$)Alkyl bedeuten,

dadurch gekennzeichnet, daß man eine Verbindung der Formel

$R^1$$SO_2$Cl     (II)

in Gegenwart von etwa äquimolaren Mengen einer Hilfsbase mit etwa äquimolaren Mengen einer Verbindung der Formel

$R^2$-$NH_2$     (III)

in einem halogenierten aromatischen Lösungsmittel umsetzt, anschließend zur Neutralisation des Hydrochlorids der Hilfsbase das Reaktionsgemisch mit wäßrigem Alkalihydroxid oder mit Alkoholaten umsetzt und die Hilfsbase abdestilliert.

$R^1$     bedeutet in obengenannten Formeln vorzugsweise ($C_1$-$C_6$)-Alkyl, insbesondere Methyl oder Ethyl.

$R^2$     bedeutet vorzugsweise Methyl oder Ethyl.

Die Verbindungen der Formel (I) sind wertvolle Zwischenprodukte bei der Herstellung von Sulfonylharnstoffen mit herbizider Wirkung (US-PS 4,169,719; EP-A 071958 = US-A 4,492,598).

Die Herstellung der Verbindungen der Formel (I) durch Umsetzung von Sulfonsäurechloriden der Formel (II) mit Ammoniak oder Aminen ist bereits bekannt.

Diese bekannten Verfahren werden jedoch in Gegenwart von wäßrigen, alkoholischen, benzolischen oder etherischen Lösungsmitteln bei tiefen Temperaturen ($\leq$ 0°C) und einem großen Überschuß (> 3 Moläquivalenten) an Aminkomponente durchgeführt (s. Houben-Weyl, Methoden der organischen Chemie, Band IX, S. 398-400, 424-425 und 605-622; Baxter et al., J. Chem. Soc. 1955 S. 669 und 670; Asinger, Chem. Ber. 75 B, 40 (1942). Bei diesen Verfahren wird die Amin-Komponente immer in großem Überschuß eingesetzt, um die Bildung von Nebenprodukten (Entstehung von Sulfimiden und sulfonsauren Ammoniumsalzen) zu unterdrücken.

Nur bei großem Aminüberschuß können bei diesen Verfahren befriedigende Ausbeuten erzielt werden.

Die Verwendung eines hohen Amin-Überschusses bringt jedoch große ökonomische und ökologische Nachteile mit sich, da überschüssiges Amin nach dessen Neutralisation abgetrennt werden muß oder das Hydrochlorid des Amins durch Extraktion oder Filtration isoliert und entsorgt werden muß.

Extraktionen und Filtrationen werden zusätzlich durch die hohe Löslichkeit der Produkte I im wäßrigen Medium und durch die hohe Löslichkeit der Amin-Hydrochloride der Hilfsbase im Produkt I (100 g Hydrochlorid der Hilfsbase lösen sich in 1000 g Produkt I) kompliziert.

Dies bedeutet einen zusätzlich technischen Aufwand.

Erhebliche Abluft- oder Abwasserprobleme sind zu bewältigen. Pro Mol überschüssiges Amin wird ein zusätzliches Mol Säure benötigt, das ein Mol Salz im Abwasser liefert (Houben-Weyl, Band IX S. 606).

Ferner ist eine Kühlung während der Reaktion notwendig, da andernfalls die Ausbeuten deutlich absinken, S. Pantlitschko, Monatshefte 89 (1958) S. 285-287; Field, Grunwald, J. Am. Chem. Soc. 75 (1953) S. 936.

Ferner ist die Umsetzung von Aminen mit Sulfochloriden in Gegenwart von Nitroalkanen bekannt (DE-OS 1 929 295). Die Umsetzung wird mit einem leichten Überschuß an Aminkomponente durchgeführt.

Wegen der hohen Toxizität der Nitroalkane ist jedoch eine Durchführung des Verfahrens im technischen Maßstab sehr problematisch.

Aus wirtschaftlichen Gründen wäre der Einsatz von Nitrolkanen in einem technischen Verfahren aufgrund der hohen Gestehungskosten dieses Lösemittels und der aufwendigen Aufarbeitungen (DE-OS 1929295 = USA 3,574,740) sehr ungünstig.

Überraschenderweise werden beim erfindungsgemäßen Verfahren die Sulfonamide der Formel (I) in quantitativen Ausbeuten und Reinheiten von über 99 % erhalten, obwohl nur etwa die äquimolaren Mengen an Amin eingesetzt werden.

Das erfindungsgemäße Verfahren zeichnet sich durch eine hohe Selektivität und hohe Reaktionsgeschwindigkeit aus.

Das Molverhältnis II:III beträgt ca. 1:1 mit Abweichungen von bis zu 10 %, die technisch bedingt sind.

Die Reaktionstemperaturen für die Umsetzung der Komponenten II und III liegen zwischen 0 und 100°C, vorzugsweise zwischen 10° und 70°C.

Die bei dem erfindungsgemäßen Verfahren verwendeten Hilfsbasen dienen zur Neutralisation des als Nebenprodukt sich bildenden Chlorwasserstoffs. Als Hilfsbasen können organische Basen wie z.B. organische Amine, beispielsweise Triethylamin verwendet werden. Vorzugsweise wird die Aminkomponente III als Hilfsbase benutzt.

Als halogenierte aromatische Kohlenwasserstoffe können z.B. Chlorbenzol oder Dichlorbenzol eingesetzt werden.

Die Produkte der Formel I besitzen in den erfindungsgemäß einzusetzenden Lösungsmitteln unerwartete schlechte Löslichkeiten: sie fallen daher als in der Reaktionsmischung suspendierte Öle an. In 1000 ml Chlorbenzol lösen sich nur ca. 15-20 g $CH_3SO_2NHCH_3$ bei Raumtemperatur.

Die sich während der Reaktion bildenden Hydrochloride der Hilfsbasen fallen als Dispersion an, so daß sich eine heterogene 3-Phasen-Mischung bildet. Aus diesem Gemisch wären die Hydrochloride nur mit großem technischen Aufwand isolierbar.

Es wurde jedoch eine andere Aufarbeitungsmethode gefunden, bei der die Hilfsbase quantitativ zurückgewonnen werden kann. Hierzu wird das Hydrochlorid mit Alkalihydroxid oder Alkoholaten, z.B. Alkalialkoholaten, gegebenenfalls in entsprechender alkoholischer Lösung eingesetzt, neutralisiert und das freigesetzte Amin direkt aus dem Reaktionsgemisch abdestilliert.

Die Aufarbeitung wird in der Weise durchgeführt, daß man in die heterogene 3-Phasenmischung die etwa äquimolare Menge an Alkalilauge bzw. Alkoholaten bei Temperaturen zwischen 0 und 100°C eindosiert und durch Temperaturerhöhung bis auf die Rückflußtemperatur des Lösungsmittels die Hilfsbase aus den Hydrochloriden freisetzt und abdestilliert.

Nach vollständiger Entfernung der Hilfsbase durch Destillation aus der Reaktionsmischung wird das Wasser bei Rückflußtemperaturen durch Auskreisen abdestilliert bzw. der Alkohol abdestilliert.

Überraschenderweise werden die an sich hydrolyseempfindlichen Sulfonamide der Formel I (vgl. Houben-Weyl, Bd. IX S. 398, 3. Absatz v.unten) nicht hydrolysiert, obwohl hohe Reaktionstemperaturen angewendet werden.

Da die Sulfonamide I stärkere Säuren als die Hydrochloride der Hilfsbase sind (Methansulfonsäure-N-methylamid: pKs = 9,3 /Methylaminhydrochlorid: pKs = 10,6), war zu erwarten, daß anstelle der Hydrochloride die Sulfonamide I bevorzugt mit der Alkalilauge oder Alkalialkoholaten unter Bildung von Sulfonamidsalzen reagieren.

Trotz der starken pks-Unterschiede reagiert jedoch nur das Hydrochlorid mit dem zugesetzten Alkalihydroxid bzw. Alkoholat, wobei etwa nur die äquimolaren Mengen an Hydroxid bzw. Alkoholat benötigt werden. Die Hilfsbasen und die Sulfonamide I fallen in nahezu quantitativen Ausbeuten und Reinheiten > 99 % an.

Für technische Umsetzungen können die Rohsuspensionen der Verbindungen der Formel (I) in Chlorbenzol, die noch Alkalichlorid enthalten, direkt ohne vorherige Reinigung für weitere Umsetzungen verwendet werden (z.B. Umsetzung mit Chlorsulfonylisocyanat zur Herstellung von Herbiziden), oder vom Alkalichlorid, vorzugsweise heiß, abfiltriert werden.

Die Hilfsbasen können nach der Aufarbeitung erneut in die Reaktion eingesetzt, also quantitativ recyclisiert, werden.

Die Lösungsmittel sind ebenfalls nahezu quantitativ recyclisierbar.

Das erfindungsgemäße Verfahren wird zweckmäßigerweise so durchgeführt, daß man das Amin der Formel (III) in die Lösung der Verbindungen der Formel (II) eindosiert. Ebenso kann jedoch auch die Amin-Komponente zusammen mit dem Lösungsmittel vorgelegt und das Sulfochlorid in diese Lösung eindosiert werden. Möglich ist ferner die simultane Zugabe der Komponenten II und III in das Lösungsmittel.

Es ist zweckmäßig, wenn auch nicht unbedingt erforderlich, das Verfahren unter Inertgasatmosphäre, beispielsweise unter Stickstoff auszuführen, um störende Einflüsse von Sauerstoff auf die Reaktion zu vermeiden.

Das erfindungsgemäße Verfahren kann kontinuierlich als auch diskontinuierlich durchgeführt werden. Es wird durch nachfolgende Beispiele erläutert:

Beispiel 1

Methansulfonsäure-N-methylamid

In einem 10-Liter Vierhalskolben werden zu einer Lösung von 4000 ml Chlorbenzol und 458 g Methansulfonchlorid bei 20-25°C über einen Zeitraum von 3-4 Stunden insgesamt 251 g Methylamin eingegast.

Nach beendeter Reaktion wird für 3 Stunden nachgerührt. Bei 25°C werden 321 g 50 %ige Natronlauge eingetropft und für 2 Stunden auf 100°C erhitzt.

Während dieser Zeit werden insgesamt 125 g Methylamin abdestilliert und in einer Kühlfalle aufgefangen. Danach wird das Wasser bei Rückflußtemperatur am Abscheider ausgekreist. Das Lösungsmittel wird im Vakuum abdestilliert.

Nach Abdekantieren vom Natriumchlorid erhält man 438 g Rohprodukt mit einer Reinheit von 99,0 %, entsprechend einer Ausbeute von 99 % d. Th.; $n_D^{22}$: 1,4509.

Beispiel 2

Ethansulfonsäure-N-methylamid

In einem 2-Liter Vierhalskolben werden in 2000 ml Chlorbenzol 125 g Methylamin vorgelegt und bei 20-25°C 257 g Ethansulfochlorid zugetropft.

Nach beendeter Reaktion wird für 2 Stunden nachgerührt. Bei 25°C werden 321 g 25 %ige Natronlauge eingetropft und für 2 Stunden auf Rückflußtemperatur des Reaktionsgemisches erhitzt.

Während dieser Zeit werden insgesamt 62 g Methylamin abdestilliert und in einer Kühlfalle aufgefangen. Danach wird das Wasser bei Rückflußtemperatur am Abscheider ausgekreist.

Das Lösungsmittel wird im Vakuum abdestilliert. Nach Abdekantieren vom Natriumchlorid erhält man 247 g Rohprodukt mit einer Reinheit von 99,6 %. Nach Dünnschichtdestillation werden 242 g destilliertes Produkt von 99,8 % Reinheit erhalten, entsprechend einer Ausbeute von 98,4 %; $n_D^{25}$ = 1,4530.

Beispiel 3

Methansulfonsäure-N-ethylamid

In eine Lösung von 400 ml Chlorbenzol werden unter Stickstoffschutzgasatmosphäre bei 20-30°C über einen Zeitraum von 4 Stunden gleichzeitig 458 g Methansulfochlorid und 360 g Ethylamin eindosiert. Nach beendeter Reaktion wird für 3 Stunden nachgerührt und mit 642 g 25 %iger Natronlauge versetzt. Das Ethylamin wird innerhalb von 2 Stunden bei Rückflußtemperatur der Reaktionsmischung freigesetzt und abdestilliert. Nach Abdestillation des Ethylamins (178 g) wird bei Rückflußtemperatur des Reaktionsgemisches das Wasser (480 g) ausgekreist.

Nach Abdestillation werden 4493 ml einer chlorbenzolischen Lösung von Methansulfonsäure-N-ethylamid erhalten, die 493 g des Produktes mit einer Reinheit von 99,7 % enthält.

Beispiel 4

Methansulfonsäure-N-methylamid

In einem 10-Liter Vierhalskolben werden in 2000 ml Chlorbenzol 250 g Methylamin eingegast. In diese Lösung werden bei 40°C unter Inertgasatmosphäre innerhalb von 4 Stunden 458 g Methansulfonsäurechlorid eindosiert. Nach beendeter Reaktion wird für 2 Stunden nachgerührt und mit 642 g 25 %iger Natronlauge versetzt und für 2 Stunden unter Rückflußbedingungen 125 g Methylamin abdestilliert.

Danach wird das Wasser bei Rückflußtemperatur am Abscheider ausgekreist. Das Lösungsmittel wird im Vakuum abdestilliert.

Nach Abdekantieren von Natriumchlorid erhält man 436 g Rohprodukt mit einer Reinheit von 99,2 %; $n_D^{22}$: 1,4510.

Beispiel 5

Methansulfonsäure-N-methylamid

In einem 10-Liter Vierhalskolben werden zu 2000 ml Chlorbenzol 458 g Methansulfonsäurechlorid unter Inertgasatmosphäre getropft. In diese Lösung werden zwischen 10-40°C innerhalb von 2 Stunden 260 g Methylamin eingegast. Nach 2 Stunden Nachrühren wird mit 660 g 25 %iger Natronlauge versetzt und unter Erhitzen der Reaktionsmischung auf Rückfluß 135 g Methylamin abdestilliert. Der leichte Natronlaugeüberschuß wird mit verd. Salzsäure zurückgewonnen.

Danach wird das Wasser während 2 Stunden bei Rückflußtemperatur ausgekreist.

Nach Abdestillieren des Chlorbenzols und Abdekantieren von Natriumchlorid werden 437 g Rohprodukt mit einer Reinheit von 99,5 % erhalten; Ausbeute: 99,5 %; $n_D^{22}$: 1,4512.

Analog zu den in den Beispielen 1-5 beschriebenen Verfahrensweisen lassen sich beispielsweise folgende Verbindungen der Formel (I) herstellen:

| Beispiel | $R^1$ | $R^2$ |
|---|---|---|
| 6 | $CH_3$ | $C_3H_7$ |
| 7 | $C_2H_5$ | $C_2H_5$ |
| 8 | $C_3H_7$ | $CH_3$ |
| 9 | $CH_3$ | H |
| 10 | $C_2H_5$ | H |
| 11 | $Cl-CH_2$ | H |
| 12 | $Cl-CH_2-CH_2$ | $C_3H_7$ |
| 13 | $CH_3O-CH_2$ | $C_2H_5$ |
| 14 | $Cl-CH_2$ | $CH_3$ |
| 15 | $C_2H_5$ | $C_3H_7$ |

Vergleichsbeispiele

A Herstellung von Methansulfonsäure-N-methylamid Gemäß Houben-Weyl, Band IX S. 606

Zu 458 g Methansulfonsäurechlorid wird bei 0°C innerhalb von 3 Stunden eine gekühlte Lösung von 250 g Methylamin in 3000 ml Ether gegeben. Nach einer Nachrührzeit von 10 min bei 0°C wird konz. Salzsäure im gleichen Volumen Wasser hinzugegeben und das Lösungsmittel anschließend abdestilliert. Der verbleibende ölige Rückstand wird in Essigester aufgenommen, das wäßrige Filtrat zweimal mit Essigester ausgeschüttelt und die vereinigten org. Phasen destilliert.

Man erhält 344 g destilliertes Produkt mit einer Reinheit von 97,8 %. Ausbeute: 76,9 %.

B Herstellung von Methansulfonsäure-N-methylamid Gemäß Baxter et al., J. Chem. Soc. 1955, S. 670

Eine Lösung von 458 g Methansulfochlorid in 3000 ml trockenem Ether wird langsam bei 0°C zu einer Lösung von 375 g Methylamin in 2000 ml Ether gegeben. Die Reaktionsmischung wird für eine Stunde bei Raumtemperatur nachgerührt, das entstandene Methylaminhydrochlorid wird abfiltriert.

Der Filterrückstand wird mit Ether und Chloroform gewaschen. Man erhält 229,5 g Methylaminhydrochlorid (85 %). Nach Destillation werden 384 g Produkt mit einer Reinheit von 97,9 % erhalten; Ausbeute: 86 %.

Vergleichsbeispiel A zeigt, daß bei Einsatz von äquimolaren Mengen Amin in den bekannten Verfahren nur unbefriedigende Produktausbeuten erhalten werden. Die Ergebnisse erfüllen den technischen Standard nicht. Selbst bei Einsatz von größeren Mengen an Amin, s. Vergleichsbeispiel B, liefern die bekannten Verfahren das gewünschte Produkt in nur ca. 85 %iger Ausbeute.

C Herstellung von Methansulfonsäure-N-methylamid in Gegenwart von Wasser ohne Abdestillieren der Hilfsbase

In einem 10-1-Vierhalskolben werden zu einer Mischung von 3000 g Chlorbenzol und 1500 g Wasser 251 g Methylamin gasförmig eingeleitet. Bei 20 bis 25°C werden innerhalb von 3 bis 4 Stunden 458 g Methansulfonsäurechlorid zugetropft, wobei der pH-Wert durch Zugabe von Natronlauge zwischen 7 und 9 eingestellt wird. Nach beendeter Zugabe läßt man noch 3 Stunden nachrühren und trennt die organische Phase ab. Nach Abdestillieren des Chlorbenzols verbleiben 62 g Rohprodukt mit einem Gehalt von 95,8 % an Methansulfonsäure-N-methylamid, was einer Ausbeute von 25,8 % d. Th. entspricht.

**Patentansprüche**

1. Verfahren zur Herstellung von Verbindungen der Formel (I)

$R^1\text{-}SO_2NH\text{-}R^2$     (I)

worin

$R^1$     $(C_1\text{-}C_6)$Alkyl, $(C_2\text{-}C_6)$Alkenyl oder $(C_2\text{-}C_6)$Alkinyl, die gegebenenfalls durch Halogen, $(C_1\text{-}C_4)$-Alkoxy oder $(C_1\text{-}C_4\text{-}$Alkoxy)-carbonyl substituiert sind, und

$R^2$     Wasserstoff oder $(C_1\text{-}C_4)$Alkyl bedeuten,

dadurch gekennzeichnet, daß man eine Verbindung der Formel

$R^1SO_2Cl$     (II)

in Gegenwart von etwa äquimolaren Mengen einer Hilfsbase mit etwa äquimolaren Mengen einer Verbindung der Formel

$R^2\text{-}NH_2$     (III)

in einem halogenierten aromatischen Lösungsmittel umsetzt, anschließend zur Neutralisation des Hydrochlorids der Hilfsbase das Reaktionsgemisch mit wäßrigem Alkalihydroxid oder mit Alkoholaten umsetzt und die Hilfsbase abdestilliert.

2. Verfahren gemäß Anspruch 1, dadurch gekennzeichnet, daß man als Hilfsbase eine Verbindung der Formel (III) einsetzt.

3. Verfahren gemäß Anspruch 1 oder 2, dadurch gekennzeichnet, daß man als Lösungsmittel Chlorbenzol oder Dichlorbenzol oder deren Gemische einsetzt.

4. Verfahren gemäß einem oder mehreren der Ansprüche 1 bis 3, dadurch gekennzeichnet, daß die Reaktionstemperatur für die Umsetzung der Komponenten II und III zwischen 0° und 100°C liegt.

5. Verfahren gemäß einem oder mehreren der Ansprüche 1 bis 4, dadurch gekennzeichnet, daß die Reaktionstemperatur für die Umsetzung der Komponenten II und III zwischen 10° und 70°C liegt.

6. Verfahren gemäß einem oder mehreren der Ansprüche 1 bis 6, dadurch gekennzeichnet, daß man zur Neutralisation des Hydrochlorids der Hilfsbase das Reaktionsgemisch bis zur Rückflußtemperatur des Lösungsmittels erhitzt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 371 271 (PENNWALT CORPORATION)<br>* Seite 2, Zeile 1 - Seite 3, Zeile 9 *<br><br>----- | | C07C303/38<br>C07C311/03<br>C07C311/24 |
| | | | **RECHERCHIERTE SACHGEBIETE (Int. Cl.5 )**<br><br>C07C |

Der vorliegende Recherchenbericht wurde für alle Patentansprüche erstellt

| Recherchenort | Abschlußdatum der Recherche | Prüfer |
|---|---|---|
| OEN HAAG | 22 JUNI 1992 | ZAROKOSTAS K. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
......................................................................
& : Mitglied der gleichen Patentfamilie, übereinstimmendes
    Dokument